# EUROPEAN PATENT SPECIFICATION

(11) **EP 1 162 948 B1**
(45) Date of publication and mention of the grant of the patent: **23.11.2005**
(21) Application number: 00911240.0
(22) Date of filing: 16.03.2000
(51) Int. Cl.: A61K 9/10

(54) **AN IMPROVED REVERSIBLE CONTRACEPTIVE FOR MALE AND FEMALE**
EIN VERBESSERTES REVERSIBLES VERHÜTUNGSMITTEL FÜR MÄNNER UND FRAUEN
CONTRACEPTIF REVERSIBLE AMELIORE MASCULIN ET FEMININ

(30) Priority: 17.03.1999 IN DE041599
(43) Date of publication of application: 19.12.2001
(73) Proprietor: Guha, Sujoy Kumar, New Delhi 110 016 (IN)
(72) Inventor: Guha, Sujoy Kumar, New Delhi 110 016 (IN)
(74) Representative: Moore, David Simon
(86) International application number: PCT/IN2000/000023
(87) International publication number: WO 2000/054746

(56) References cited:
- GB-A- 1 569 660
- US-A- 4 040 417
- US-A- 4 185 618
- US-A- 5 488 075
- CHEMICAL ABSTRACTS, vol. 109, no. 25, 19 December 1988 (1988-12-19) Columbus, Ohio, US; abstract no. 223418, REN, CAIYUAN ET AL: "Image-forming contraceptive for blocking of oviducts or vas deferens" XP002143456 & CN 85 108 504 A (NORTHWEST UNIVERSITY, PEOP. REP. CHINA) 20 May 1987 (1987-05-20)

## Description

### Technical Field of the Invention :-

The present invention relates to an improved reversible contraceptive for use by male and female, particularly it relates to an injectable reversible contraceptive consisting of contraceptive polymer, an electrically conducting material and magnetic material in a solvent medium, more particularly it relates to an injectable reversible contraceptive consisting of copolymers of styrene maleic anhydride and styrene maleic acid, and micro and macro size particles of magnetic material and electrically conducting material in pure dimethyl sulphoxide having improved contraceptive action as well as more controlled delivery method and reversal action to restore fertility and capable of imaging by ultrasound. X-ray, CAT scan, MRI and scanning electrical impedance plethysmography, and capable of better control and determination of quantum of distribution within the reproductive ducts by external magnetic field.

### Background Art of the Invention :-

The progeny is formed by the union of the male sperms and the female ovum, a process known as fertilization. In the male the sperms travel from the testes via the epididymis along a tube known as vas deferens. In the female the ovum comes down the tube known as fallopian tube also as the oviduct and the sperms from the male travel up this same tube and fusion of the sperm and the ovum takes place in the fallopian tube. If by surgical means or placement of a contraceptive device or a preparation in the male vas deferens, the biological activity of the sperm is significantly altered then the sperms will not be able to effectively fuse with the ovum and produce a progeny. Similarly, if by the surgical means or placement of a contraceptive device or a preparation in the female fallopian tube a significant alteration in either the sperm or the ovum or both is caused, then the fusion of the sperm and the ovum is prevented and fertilization followed by progeny development does not take place. Various surgical, means and contraceptive devices and preparations have been developed over last few decades to avoid fertilization followed by progeny development.

One of the known surgical means, which is commonly used and known as vasectomy consists of cutting and tying the vas deferens in males. Similar operation in the female is known as tubectomy, which involves cutting and tying of the fallopian tube in female. This surgical procedure results in prevention of flow of sperms through vas deferens in males and flow of ovum through fallopian tube in females in forward direction. The major disadvantage of vasectomy is that it is surgical in nature and the reversal of the fertility is not effective. The rejoining or opening of vas deferens or of fallopian tube does not result in the desired fertility, because the level of generation of antibodies even after rejoining or opening of vas deferens remains high, which in turn continue to destroy the sperms. In the female rejoining or opening of the fallopian tube often produces inadequate transport of ovum.

Another class of known contraceptives in the art for the male includes repeated administration of androgens, such as testosterone enanthate. A regimen of weekly intramuscular injection of 200 mg of testosterone enanthate was tried in men [*Lancet (1990) 338 (8721) : 955-959*]*.* The trials in animals resulted azoospermia or oligospermia in most of the animals [*Fertil. Steril. (1977) 28 : 1320-1328*], and trials in men resulted in azoospermia in about 55% of subjects with oligospermia observed in a further 40%. Other side effects included weight gain, greasiness of skin and lowering of high density serum lipoproteins, which are thought to be cardioprotective [*J.Clin.Endocrinol.Metab. (1991) 73 : 4-7*]. An attempt to find more effective androgens resulted in development of a drug 17a-beta-hydroxy-7-alpha-methyl-D-homo-19-norandrost-4, 16-diene-3-one and its 17a-beta hydroxy esters (*US Patent no. 4,788,218*). These were found to be active by subcutaneous injection, maintaining libido, however, consistent reduction in spermatogenesis could not be obtained. Further research led to development of long acting esters of testosterone, like testosterone buciclate with the general formula Tes-CO-R-R' (*US Patent no. 4,948,790*)*,* which have shown high effectiveness in animals to suppress spermatogenesis. Although, these drugs held promise, results from various trials indicated that periodic administration of supra-physiological doses of androgens alone led to wide fluctuations in androgen levels and hence sustained release methods were advocated.

Still another class of known contraceptives in the art is combination of androgens with progestagens, which have shown certain advantages over the androgens alone [*Fertil. Steril. (1989) 52(6):1011-1018 and Fertil. Steril. (1993) 60(6):1062-1068*]. Even studies of this class of contraceptives indicated some problems, such as need for quite high doses of gestagen and adverse side effects on account of the oestrogenic activity of the gestagen. Yet another class of known contraceptives in the art is of gonadotrophin releasing hormone (GnRH) or luteinising hormone releasing hormone (LHRH) [*New Engl. J.Med.* (*1981*) *305 : 663-667*], which due to accompanied decreased libido and potency suggested a need for addition of testosterone. Further, some of the agonists of LHRH are resistant to biodegradation. Therefore, the LHRH agonist approach is no longer being pursued. Instead, LHRH antagonists, the another class of the known contraceptives appeared to be very promising. Quite a large number of these antagonists have been made available (*US Patent no. 5,171,835*) and [*J.Clin.Endocrinol.Metab. (1993) 77(2):427-432*]*.* Such antagonists suffer from the limitation of requirement of judicious balance between testosterone suppression and supplementation. It is unlikely that a fixed dose schedule effective in all men will ever be realised and hence their practical utility will probably be limited.

Further class of known contraceptives in the art was of anti-androgens, which were studied for their contraceptive action [*Contraception (1976) 14:403-407*]. Cyproterone acetate (CPA), which was administered orally and from subcutaneous implants, was thought at that time to inhibit supermatozoan maturation in the epididymis. However, on account of side effects, especially libido reduction, at the doses necessary to induce azoospermia or oligospermia, this compound and anti-androgens as such were virtually given up. Now combination of oral cyproterone acetate with testosterone like injectables are being assessed, but duration of effectiveness and efficacy in the people of all ethnic origin is not satisfactory.

Still further class of known contraceptives in the art was of follicle stimulating hormone (FSH) inhibiting proteins (*US Patent No. 5*,*015*,*729 and 5,037,805*). Although, these drugs have shown promising results during clinical trials, but the outcome is very species specific and effective long-term immunogenicity seems difficult. Hence, despite advances in the synthetic chemistry of human inhibin, the possible role in human fertility control is far from clear. None of the immunological drugs have undergone long-term trials.

Yet another class of known contraceptives in the art is of astringents like zinc and tannins (*US Patent No. 4,156,427*) and glycerol (*US Patent No. 4*,*720*,*507*). Such contraceptives are directly injected into testes, which often results in testicular atrophy and generally leads to permanent sterilization with no scope of reversal, even with surgical intervention. Practical drugs for reversible contraception on this basis are yet to be tried.

Still another known contraceptive in the art consists of un-complexed microsize particles of copper of size between 0 to 90µm alone taken in castor oil, which is injected into the epididymis in a 'blind approach' [*Int.J.Andrology (1985) 8:168-174*]*.* The major disadvantage of such use of un-complexed microsize particles of copper in castor oil is that the contraceptive in part enters in the epididymal tubule and more so remain outside the tubule and therefore produces histological damage to the epididymis and there being no control on backtracking of the contraceptive preparation into the testes and hence the testicular tissue is adversely affected. By this means the contraception is achieved in majority of animals tested but not in all and further along with the contraceptive effect the adverse effects on account of tissue damage occurs. Furthermore, there is no scope of removal of the contraceptive preparation for restoration of fertility. Yet another known contraceptive in the art consists of exclusively un-complexed microsize particles of copper, which are neither injected in the vas deferens or epididymis nor in the fallopian tube but are inserted in the wall of the vas deferens by means of iontophoresis of copper [*Andrologia (1982) 14:481*]. This contraceptive has short lived contraceptive effect and the planned removal of the contraceptive from the wall of the vas deferens to restore fertility prior to self reversal time is not possible.

Still another class of known contraceptives is in the form of contraceptive device, which can be inserted in the vas deferens or the fallopian tube. The major disadvantage of the contraceptive devices is that generally these are non-reversible with some exceptions.

One class of known contraceptive devices in the art is of those using copper in its wire form only. Such contraceptive devices include contraceptive device consisting of copper in wire form only, which is inserted into lumen of the vas deferens [*Contraception (1994) 29: 45-48*] or contraceptive device consisting of copper in wire form only, which is inserted into the lumen of the fallopian tube [*Indian J.Exp. Biol. (1976) 14:316-319*]*.* The major disadvantage of using such contraceptive devices consisting of copper in wire form only is that such form of copper is fairly stiff structure and generally causes injury to the vas deferens or the fallopian tube, as the case may be and may often cause puncture, which may result in irreversible damage to the vas deferens or the fallopian tube. Further disadvantage of using wire form of copper as contraceptive device in male or female is that such form of copper may subsequently cause fibrosis, which makes the removal of the wire form of copper device difficult with very low return of fertility.

Another class of contraceptive device known in the art, which uses copper or its alloy in its coil form only is of a contraceptive device consisting of the copper or its alloy only in the form of coil, which is given a shape to have larger surface area and is anchored within the fallopian tube by a lumen traversing region of the resilient structure which has a helical outer surface, together with a portion of the resilient structure which is biased to form a bent secondary shape, the secondary shape having a larger cross-section than the fallopian tube. The resilient structure is restricted in a straight-configuration and transcervically inserted within the fallopian tube, where it is released [*Patent Application no. WO 99*/*15116, date of publication 10.08.99*]. Still another known contraceptive device consists of copper in its ring form disposed between the ribs of the elongated tubular member or as coating over the rib structure of the elongated tubular member having a central lumen and a flange formed at the proximal end. Such device is formed from the plurality of flexible ribs configured to provide a plurality of seals within the interstitial portion of a fallopian tube and is provided with a valve member within the lumen of the tubular member (*US Patent no. 5*,*935*,*137*, *date of publication 01.04.99*)*.* Such devices also suffer from the disadvantages of being fairly stiff structure and of complicated structure to be fabricated and generally causing injury to the fallopian tube, which may be irreversible in nature and may consequently cause fibrosis. These devices result in permanent sterilization with no scope of reversal of fertility. Further these devices are restricted for use by female only.

Still further class of known contraceptive devices or methods in the art is to affect and/or block the flow of sperms in the vas deferens or ovum in fallopian tube and such contraceptives consists of implantation of a reversible occulusion contraceptive device. Such known devices are generally suitable for contraception or sterilization either of male or of female and not of both.

A class of known surgical contraceptive or occlusion devices, which are suitable for use by male includes an elongated member provided with annular flange (*US Patent no. 3*,*828*,*764*), proximal or a distal tube (*US Patent no. 3*,*990*,*434*), an elongated hollow tube provided with an elastic cap and a plug (*US Patent no. 4,682,592*)*,* a filament (*US Patent no. 5,471,997*)*,* an expandable body (*Patent application no. WO 97*/*16132*), urethral sealing member (*US Patent no. 5,603,335*) and urethral occlusive device (*US Patent no. 5*,*884*,*629*). The elongated member provided with an annular flange is inserted into severed ends of vas by cutting the vas and sutured to one or both ends of the vas to prevent migration of the device in the vas and is provided by transverse wall to block fluid flow through the vas (*US Patent no. 3*,*828*,*764*). The reversibility of fertility is achieved by surgically removing the transverse wall. The proximal tube having a shoulder portion and openings on either end is inserted in the vas after puncturing by hypodermic needle and a distal tube similar in construction to the proximal tube is also inserted in the vas after making second puncture in the vas along with a closed plug for the prevention of passage of body fluid (*US Patent no. 3,990,434*). The reversibility of fertility is achieved by surgically replacing the closed plug with an open plug. A further development of contraceptive device led to the development of a contraceptive device comprising an elongated hollow tube having an expandable elastic cap forming a fluid tight seal at one end and a plug, including a valve, forming a fluid tight seal at the other end and also a flexible spring within the said tube allows selective ingress and egress of the fluid (*US Patent no. 4*,*682*,*592*). This device prevents the transport of sperms by occluding the lumen of vas, the ejaculatory duct or the urethra and is inserted through the urethra. A filament having length at least about one fourth of length of vas and outside diameter about equal to inside diameter of vas and containing an enlargement at one end and is made of a material inert to tissue is inserted in the vas after making an insertion in one wall of vas near the epididymis (*US Patent no. 5*,*471*,*997*). This device does not block the passage of the sperms but allow escape of sperms from vas. An expandable body unit having an elastic member, an opening cavity and first, second and third progressively rearwardly spaced cavity regions, a cam member and forwardly extending shaft is inserted into a male urethra penis cavity for blocking the urethra (*Patent Application no. WO 97*/*16132*). This device is provided with the facility for preventing its successive uses. The containment type inboard contraceptive device including a urethral sealing member in the form of an oblong ring and flexible container bag and a tail or other tensioning means attached to the bag to regulate position of the device is inserted into the urethra in the penis to block sperm passage (*US Patent no. 5,603,335*)*.* The fertility is restored by not using the device. An urethral occlusive device is designed for insertion into male urethral opening (*US Patent no. 5*,*884*,*629*).

Another class of known surgical contraceptive or occlusion devices, which are suitable for use by male and female includes multifunctional surgical device (*US Patent no. 4*,*788*,*966*), sterilization clip (*US Patent no. 5*,*193*,*554*), stent (*US Patent no. 5,474,089*) and an occluding member comprising a tubular framework (*Patent Application no. WO 98*/*26737*)*.* A multifunctional surgical device is designed to apply an elastic occluding ring onto an anatomical tubular structure or for cutting and cauterizing the cut ends of such a structure or for applying a conventional clip to such a structure (*US Patent no. 4*,*788*,*966*). A sterilization clip comprising of an upper and lower jaw made-up of plastic material with capture means for capturing the fallopian tube or vas deferens and such capture means are provided with soft lining blocks the passage of sperms or ovum by compressing the vas deferens or the fallopian tube (*US Patent no. 5*,*193*,*554*). A stent including an expandable section and predetermined portion which is ablatable by application of laser irradiation and such portion includes a guiding segment to guide a fiber optic device or alternately stent includes a collapsible frame structure compressed by a spring, and a central ablatable blocking portion is non-surgically inserted (*US Patent no. 5*,*474*,*089*). The reversibility is achieved by applying a laser beam to ablate the portion of the blocking device in order to reopen the duct and to re-establish fertility. An occluding member comprising a tubular framework formed from a shape memory material is configured to be implanted in a reproductive lumen and secured to the wall thereof, alternatively, the occluding member may be collapsed upon a solid plug (*Patent Application no. WO 98*/*26737*). The reversibility is achieved by reopening tubular framework by introducing a balloon catheter and by series of inflations of the balloon re-expanding the collapsed occluding member or by removing the plug.

The above two classes of known contraceptive devices and their methods of use, as described herein above for use by male or male and female, suffer from one or more of the disadvantages or limitations. One such disadvantage associated with such know devices and method of use thereof is that such known devices simply act as a blocking device to the passage of sperm and/or ovum. Although contraception or sterilization is obtained for some time, the block also prevents the movement of material other than the sperm and/or ovum as for example water and/or proteins. Hence there is pressure buildup, which may result in damage to the epididymis in the male and ovary in the female. Another disadvantage of such known devices is imperfect blockage, which in turn results in imperfect destruction of the sperm and/or ovum. Still another disadvantage of some of such known devices is that such devices have no anti-sperm and/or anti-ovum action, so sperm and/or ovum leaking past the device when the vas deferens lumen and/or fallopian tube dilates can produce pregnancy. Yet another disadvantage of such known devices is that the use of such devices calls for surgical implantation, which is a cumbersome procedure. Further disadvantage of such known devices is that such devices can get displaced and pregnancy may occur. Still further disadvantage of such known devices is that the restoration of fertility using such devices requires the surgical exploration and hence the success rate will be low. Yet further disadvantage of such known devices is that the initial total blockage leads to rise in anti-sperm antibody titer in the male and retrograde actum on the ovary in the female, which reduces the chance of actual fertility restoration even when by surgery the device is removed or replaced by another such device for restoring fertility. Still another disadvantage of such known devices is that the insertion into the urethra of some of such devices either permanently, till desired by the patient or temporarily, for each act of intercourse, is painful and likely to produce erosion and infection. Yet another disadvantage of such known devices is that the use of some of such devices requires attention of the user prior and after such intercourse. Further disadvantage associated with such known devices is that the large size of some of such devices leads to rupture of the vas deferens in many cases. Still further disadvantage associated with such known devices is that the use of some of such devices totally destroys a segment of the vas deferens and/or fallopian tube, hence reversal would require special microsurgery to rejoin the vas deferens and/or fallopian tube. Yet further disadvantage associated with such known devices is that the removal of some of such devices becomes difficult and calls for a complicated procedure on account of fibrosis with low success rate. Yet another class of occlusion methods to achieve contraception or sterilization includes formation of a chemical preparation based plug in the vas deferens and/or fallopian tube, such as formation of polyurethane plug (*UK Patent no. GB2223025*) or nylon plug (CN 85108504A) or neem oil plug (*US Patent no. 5*,*501*,*855*) or styrene maleic anhydride copolymer plug (*US Patent no. 5*,*488*,*075 and Indian Patent no. 183196*). The polyurethane plug is formed by reacting pre-polymer of polyurethane with a chain-enlargement agent with amino-group under normal atmospheric temperature or above it in the presence of an organic solvent and organic acid catalyst, which is solidified in the ductus deferens (*UK Patent no. GB2223025*). The reversibility is achieved by removal of the plug. The nylon plug is formed by reacting nylon, EtOH, phenol, Cu-Zn alloys and glycerol which also results in blockage of oviduct or vas deference (CN85108504). Attention is also drawn to Chemical Abstracts, Vol 109, No. 25 (1988-12-19), Abstract No. 223418 which discloses an image forming contraceptive comprising nylon, ethanol, phenol, Cu-Zn alloys and glycerol.

The neem oil plug is formed by intra-vas administration of neem oil to male rats, which resulted in blockage of spermatogenesis without affecting the testosterone production (*US Patent no. 5,501,855*)*.* The reversibility has not been achieved. The styrene maleic anhydride copolymer plug is formed by step irradiation of styrene maleic anhydride at a dose of 0.2 to 0.24 megarad for its every 40 gms followed by dissolution in pure dimethyl sulphoxide and thereafter injected into the lumen of the vas deferens (*US Patent no. 5*,*488*,*075 and Indian Patent no. 183196*). The reversibility is achieved by flushing of the styrene maleic anhydride copolymer plug by injecting extra dose of pure dimethyl sulphoxide. The polyurethane and nylon plugs suffer from similar disadvantages as that of the contraceptive devices for use by male or by male and female, as described herein above. The neem oil plug preparation has limitation that, it does not polymerise rapidly after injection and hence travels retrograde into the testes. Further, it causes damages to the testes and testicular size reduces. Still further disadvantage of neem oil plug is that the lymph nodes are affected. Yet another limitation of the neem oil plug is that the restoration of fertility is not possible.

The styrene maleic anhydride copolymer plug, herein after referred to as SMA plug, which is undergoing multicentric Phase-III Clinical Trials in India and has been developed by the inventor of the presently disclosed invention, has been observed to have certain limitations, such as, it cannot be detected and/or suitably quantified externally by means of X-ray, CAT scanning, magnetic resonance imaging (MRI) or magnetic field due to the non-radio-opaque nature of the contraceptive drug. Another limitation of SMA plug is that, its spread or distribution in the reproductive tract after injection cannot be controlled. Further limitation of SMA plug is that, for its removal to restore the fertility, another injection of extra dose of dimethyl sulphoxide is required to be given to the patient, hence it cannot be removed from the body conveniently by non-invasive and external means except by using the reversal device disclosed in the *pending Indian Patent application no. 928*/*DEL*/*97*, developed by inventor of the present invention. However, the removal of the contraceptive even by this device is not 100% successful due to the difficulty in removal of the contraceptive from the part of vas deferens leading to the prostate region.

The limitations of SMA plug, as described herein above, have also been observed in other similar contraceptive preparations, such as polyurethane plug, neem oil etc., which are intended to be used by male or female or male and female for blockage of the vas deferens and/or fallopian tube by way of formation of plug or for affecting the nature of sperm and/or ovum for achieving the contraception or sterilization, as the basic compound of the such known contraceptive preparations being non-radio-opaque and nonmagnetic in character. The MRI also fails to detect the contraceptive mainly duc to its poor contrast with the soft tissue. The ultrasound is also incapable of detecting the contraceptive, due to low percentage of the basic compound and inadequate difference of the characteristic impedance from the body tissue. To overcome this disadvantage, if the net percentage of the basic compound is increased, it will have the disadvantages associated with the higher percentage of such basic compounds. Furthermore, some of the regions of the vas deferens and epididymis are so placed that the ultrasonic approach is not practicable.

### Need of the Invention :-

Therefore, there is a need to have a contraceptive, which can overcome all or some of the disadvantages and limitations of the prior art, as described herein above and particularly which is not only reversible in nature but also suitable for male and female subjects and as well as has improved contraceptive action and better controlled delivery, and which necessarily does not require any surgery or flushing of any solvent for its removal from the reproductive duct to restore the fertility but can be removed from the body by non-invasive and external means, and can also be imaged by X-ray, CAT scan, ultrasound, MRI and scanning electrical impedance plethysmography, and the spread or distribution of which can be controlled and quantified within the reproductive tract after injection by external means, further which is required to be injected only once to achieve the contraception.

### Aim of the Invention :-

The present invention seeks to make a complete disclosure of an improved injectable reversible contraceptive for use by male as well as by female which is not only reversible in nature but also suitable for male and female subjects and can over come some of the disadvantages and limitations of the prior art, as described herein above.

The invention also seeks to propose a contraceptive, which has improved contraceptive action as well as more controlled delivery method and reversal action to restore fertility.

The invention seeks to propose a contraceptive, which is capable of imaging by X-ray, CAT scan, ultrasound and MRI, and capable of better control and determination of quantity within the reproductive duct by external means.

Still further the present invention seeks to disclose a contraceptive which may be detected by scanning electrical impedance plethysmography.

The invention also seeks to propose a contraceptive the spread or distribution of which can be controlled in the reproductive tract after injection by external means.

This invention further seeks to disclose a contraceptive which necessarily does not require any surgery or flushing of any solvent for its removal from the reproductive duct to restore the fertility and can be removed from the body by non-invasive and external means.

This invention further seeks to disclose a contraceptive, which is required to be injected only once for achieving contraception.

This invention further seeks to disclose a contraceptive which can be reversed by external non-invasive means and necessarily does not require additional injection of an extra dose of the pure solvent, thus avoid the second injection.

This invention also seeks to disclose a contraceptive which not only acts as a blocking agent but also brings about changes in the sperm and/or ovum to result in the contraceptive action, hence overcome the disadvantages associated with the contraceptives capable of acting as blocking agent alone.

### Brief Description and Preferred Embodiments of the Invention :-

Accordingly this invention provides an injectable reversible contraceptive, and a method of preparation and use thereof, comprising a contraceptive polymer, a solvent medium, an electrically conducting material and a magnetic material, characterised in that the contraceptive polymer is from the hydrogel class of polymers, particularly a mixture of styrene maleic anhydride copolymer and styrene maleic acid copolymer, and the solvent medium is dimethyl sulphoxide solvent, and the electrically conducting material is copper in its pure form essentially consisting of microsize particles and macrosize particles, and the magnetic material is iron in its pure form essentially consisting of microsize particles and macrosize particles. In preferred forms, the invention discloses a contraceptive consisting of contraceptive polymer having electrical charge and pH lowering properties, a solvent medium having complexing properties, an electrically conducting material having charge transfer, sperm membrane and ovum covering molecule exchange, and inductive heating properties and magnetic material having magnetising and magnetic force drag properties to achieve the electrical conduction, electrical charge transfer and magnetising properties of the proposed contraceptive. Additionally the size and mechanical consistency of electrically conducting material and magnetic material are so selected that the mechanical characteristic impedance to the passage of ultrasound becomes significantly different from that of body tissue and hence the presence of the contraceptive within the body and its location can be determined by ultrasonography. Furthermore, the quantum of the presently disclosed contraceptive within the reproductive tract can be determined non-invasively by magnetic field estimations as well as by X-ray imaging, CAT scan, MRI scan and scanning electrical impedance plethysmography.

This is an additional embodiment of the present invention that in order to restore the fertility, that is to remove the contraceptive from the reproductive tract, as and when desired by the subject, the contraceptive is heated by virtue of its electrical properties by electromagnetic induction with fields from outside the body. The heating changes the basic polymer characteristics thereby lowering its contraceptive action to obtain restoration of fertility as and when desired. Further embodiment of this invention includes lowering of viscosity of the contraceptive on induction heating which further facilitates the removal of the preparation from the body by an externally imposed magnetic field, preferably travelling magnetic field so as to restore reproductive functions as and when desired.

Still further embodiment of this invention includes that the swelling and anchoring properties of the presently disclosed contraceptive without adhesion gives long term retention for the contraception with a one time administration. The contraceptive of the presently disclosed invention may also be administered after removal if the person after a period of fertility restoration desires to have contraceptive status.

Yet another embodiment of this invention includes that the contraceptive preparation when placed in the vas deferens or the fallopian tube brings about changes in the sperm and/or ovum to result in contraceptive action. The change is effected by electrical charge properties of the contraceptive polymer; and the charge transfer, and sperm membrane and ovum cover molecule exchange capabilities of the electrically conducting material.

The removal of the contraceptive from the vas deferens or the fallopian tube, in accordance to the preferred embodiment of the present invention is possible by using the magnetic properties of the contraceptive preparation to propel the contraceptive for voiding and restoration of fertility by external magnetic field or alternately is possible by flushing by another injection of the pure solvent.

The present invention therefore overcomes the disadvantages and limitations of the class of known contraceptives, which are used in the vas deferens and fallopian tube.

Further, the present invention has the advantage of electrical charge producing and pH lowering polymer, associated with the molecule exchange property and charge transfer property of the electrically conducting material, employed in the presently disclosed contraceptive preparation to greatly minimize the pressure buildup with consequent adverse tissue and immunological changes.

Still further the present invention has the advantage, that the contraceptive is controlled *insitu* by the application of a drag force or a propelling force by means of an external magnetic field. The present invention also discloses the property of residual magnetism in the contraceptive after withdrawal of the external magnetic field. Therefore, the presence of the contraceptive can be detected and to some extent can also be quantified by measuring the residual magnetic field strength from outside the body. Thus a disadvantage of the such known contraceptives that the spread in the reproductive tract after injection cannot be controlled and the presence within the body cannot be suitably located and quantified are overcome by the presently disclosed contraceptive.

### Detailed Description of the Invention :-

In accordance with this invention an improved injectable reversible contraceptive and the method of preparation and use thereof is disclosed wherein the base compound is a contraceptive polymer, which can generate an electrical charge when in contact with body water. A range of polymers can be used and a preferred class of polymers is the hydrogel class, which swells and invaginates into the folds of the lumen to help retention but does not adhere to tissue thereby allowing scope for removal. Among the hydrogel class of polymers several different polymers may be used, however the mixture of styrene maleic anhydride and styrene maleic acid copolymers is the most preferred option. In accordance to the preferred embodiment of this invention the styrene maleic anhydride and styrene maleic acid copolymers are first mixed and then dissolved in the solvent medium, preferably dimethyl sulphoxide solvent or alternately are directly dissolved in the solvent medium followed by mixing. An additive, herein referred as electrically conducting material is added to the contraceptive formulation to obtain electrical conductivity and charge transfer capabilities of the presently disclosed contraceptive. In accordance to the preferred embodiment of this invention the electrically conducting material is preferably copper in its pure form consisting of microsize particle and macrosize particle. The electrically conducting material, preferably the copper particles, as employed in the presently disclosed contraceptive, besides giving electrical conductivity also enhances the active property of the contraceptive polymer, herein referred as base polymer, to affect the structure of the sperm and the ovum by displacing some specific molecules from the sperm, for example zinc and ovum, for example proteins. In accordance to one of embodiments of this invention, when an external time varying magnetic field is applied there is electromagnetic induction and the copper particles of the presently disclosed contraceptive get hot. A microwave field is very effective in this respect and can change the polymer constitution as well as viscosity. Another additive, herein referred as magnetic material, is added to the polymer to impart the magnetic properties to the presently disclosed contraceptive. In accordance to the preferred embodiment of the present invention, magnetic material is iron in pure form or in the form of oxide or a combination with copper or with a biologically accepted material like sulphur, more preferably magnetic material is iron in its pure form consisting of microsize particle and macrosize particle. In accordance to this invention the above stated additives are uniformly dispersed in the base polymer and the aggregation of the magnetic material, preferably iron particles is prevented by suitable coating, which is preferably of cross-linked styrene maleic anhydride copolymer.

The particle size as well as the percentage by weight with respect to the contraceptive polymer of both the materials, that is electrically conducting material and magnetic material are so selected that the contraceptive action as well as the propelling functions of the contraceptive are achieved adequately. In addition these factors are controlled so that the mechanical properties at frequency of 1 to 60 MHz become significantly different from body tissue to be able to distinguish the contraceptive by its ultrasound reflection and refraction properties. Further on account of the electrical conductive property on passage of a low level alternating current the electrical impedance offered by the contraceptive preparation of this invention within the reproductive tubes will be different from that of the surrounding tissues, hence the presence of the presently disclosed contraceptive may be detected by scanning electrical impedance plethysmography also.

In accordance to the presently disclosed invention the particle size of microsize particles of electrically conducting material is about 0.005 to 20µ, preferably about 0.5 to 15µ and of macrosize particles of electrically conducting material is about 150µ to 0.2mm. Further, the particle size of microsize particles of magnetic material is about 0.005 to 15µ, preferably about 0.5 to 15µ and of macrosize particles of magnetic material is upto 0.5mm. In accordance to this invention the microsize and macrosize particles of electrically conducting, material are taken approximately in equal amounts by weight, and the microsize particles of magnetic material are taken in lower amount as compared to the macrosize particles of magnetic material. Further, in accordance with the present invention the quantum of electrically conducting material and magnetic material each varies between 3 to 20% by weight of the contraceptive polymer, particularly the electrically conducting material is taken between 3-8%, preferably between 4-6%, more preferably about 5% by weight of contraceptive polymer and magnetic material is taken between 6-15%, preferably between 8-12%, more preferably about 10% by weight of the contraceptive polymer.

In accordance to this invention the styrene maleic anhydride copolymer is prepared by the process known in the art or as disclosed in the *US Patent no. 5*,*488*,*075 and Indian Patent no. 183196* of the present inventor. The styrene maleic acid copolymer is prepared from styrene maleic anhydride copolymer either by the process known in the art or by the process disclosed herein after. For preparation of styrene maleic acid copolymer about 0.5gms of styrene maleic anhydride copolymer is taken in a round or flat bottom flask. About 50ml of about 0.5N NaOH is added to this amount of styrene maleic anhydride copolymer. The solution is left for refluxing for about 8 hrs. The refluxed material is allowed to cool down to ambient temperature followed by neutralisation with about 0.5N HCl till white precipitates of styrene maleic acid copolymer are formed. The precipitates of styrene maleic acid copolymer are separated and washed with distilled water and dried in vacuum. It is assured that styrene maleic anhydride copolymer and styrene maleic acid copolymer are free from their respective monomers.

In accordance to this invention, the presently disclosed contraceptive is prepared by dissolving the weighed quantities of styrene maleic anhydride copolymer, styrene maleic acid copolymer, electrically conducting material and magnetic material in the solvent medium, preferably in dimethyl sulphoxide followed by keeping the complex solution of the copolymers, the electrically conducting material and the magnetic material in an inert environment, preferably in nitrogen atmosphere and shaking for about 45-50 hrs by maintaining the temperature at about 35°C. The magnetic material is coated magnetic material to avoid aggregation of the magnetic particles. In accordance to preferred embodiment of this invention the copolymers, and the electrically conducting material and magnetic material are first mixed and then dissolved in the solvent. Alternately, the copolymers, and the electrically conducting material and magnetic material are directly dissolved in the solvent followed by mixing. In accordance to another preferred embodiment of this invention the copolymers are first mixed and then dissolved in the solvent followed by addition of the electrically conducting material and magnetic material. The electrically conducting material and the magnetic material are added either together or one after the other.

According to the preferred embodiment of this invention, the weighed quantities of styrene maleic anhydride copolymer, styrene maleic acid copolymer, electrically conducting material and magnetic material are first mixed together and then dissolved in about 99% pure dimethyl sulphoxide. Such manner of mixing assures the uniform distribution of particles of electrically conducting and magnetic materials.

In accordance to one of the preferred embodiments of this invention, the weighed quantities of styrene maleic anhydride copolymer, styrene maleic acid copolymer, electrically conducting material and magnetic material are directly dissolved in the solvent, preferably in about 99% pure dimethyl sulphoxide followed by mixing.

In accordance to one of the preferred embodiments of the present invention weighed quantities of styrene maleic anhydride copolymer and styrene maleic acid copolymer are first mixed and then dissolved in a solvent. To this complexed solution of contraceptive polymer and solvent medium weighed quantities of electrically conducting material and of coated magnetic material are added.

In accordance to the preferred embodiment of the present invention the weighed quantities of electrically conducting material and of magnetic material are added either together or one after the other.

In accordance to this invention the weighed ratios of styrene maleic anhydride copolymer and styrene maleic acid copolymer may be selected over a wide range to suit the specific need in terms of time period for onset of contraceptive action, duration of contraceptive action and extent of intervention required for reversal. Particularly, the ratio of styrene maleic acid copolymer and styrene maleic anhydride copolymer with respect to each other varies between 1.5:8.5 to 3:7, preferably 2:8. The scope of the present invention is not limited by the molecular weight of styrene maleic anhydride copolymer or styrene maleic acid copolymer.

Experimentally, the contraceptive of the presently disclosed invention can be prepared by mixing 70% of styrene maleic anhydride copolymer, 15% of styrene maleic acid copolymer, 5% of electrically conducting material and 10% of magnetic material and dissolving this mixed composition in about 99% pure dimethyl sulphoxide in the ratio in a manner that for every 100mg of the contraceptive polymer, that is for every 100mg of mixture of styrene maleic anhydride copolymer and styrene maleic acid copolymer about 200µl of dimethyl sulphoxide is added. This complex solution of styrene maleic anhydride copolymer, styrene maleic acid copolymer, electrically conducting and coated magnetic materials in the solvent is kept in nitrogen atmosphere and shaken for about 47 hrs by maintaining the temperature at about 35°C. The resulting viscous contraceptive preparation is placed in the syringe for injection while ensuring that atmospheric air and moisture do not come in contact with the contraceptive preparation.

In accordance to another experiment of the present invention, 80mg of styrene maleic anhydride copolymer, 20mg of styrene maleic acid copolymer, 5mg of 99.9% pure copper particles consisting of microsize and macrosize particles and 10mg of coated iron particles consisting of microsize and macrosize particles are mixed with 200µl of about 99% pure dimethyl sulphoxide. The particle sizes of copper and iron particles are maintained within the limits described herein above. This complex solution of styrene maleic anhydride copolymer, styrene maleic acid copolymer, copper particles and coated iron particles in dimethyl sulphoxide is kept in nitrogen atmosphere and shaken for about 48 hrs by maintaining the temperature at 35°C. The resulting viscous contraceptive preparation is ready for injection to the desired male or female and is placed in the syringe for injection while ensuring that atmospheric air and moisture do not come in contact with the contraceptive preparation.

The contraceptive of the present invention can be injected in male or female by any known means. However, specially designed process is described herein after merely for understanding. This described process is to take care of the specially embodied properties, particularly the charge transfer, electrical and magnetic properties of the presently disclosed contraceptive. In accordance to the preferred embodiment of the present invention the contraceptive preparation is taken in 250µl syringe, which is provided with about 23gauge needle.

In the male a puncture is made in the middle of the anterior surface of the scrotum, through which a small segment of vas deferens of the left side is delivered without injuring the vas deferens. This procedure is referred as 'no scalpel' procedure. Applying compression with the fingers onto the proximal portion of the vas deferens, that is towards the testis, the needle is inserted into lumen of the vas deferens with the needle pointing distally, that is towards the ejaculatory duct. The planned amount of the drug, which is guided by various factors, such as time period for onset of contraceptive action, duration of contraceptive action and extent of intervention required for reversal, as described herein above, is injected and typically the dose is 120µl. In order to control the distribution of the contraceptive a strong electromagnet with field strength of above 2500 gauss is placed over the surface marking the inguinal canal a little distally than the external inguinal ring. The vas deferens is then allowed to slip back into the scrotum through the scrotal puncture hole and the vas of the other side is delivered through the same hole and the procedure is repeated. The advantage of this procedure is that no suturing of the puncturing hole is required and only a strip of tape is placed over puncture hole. The subject is advised not to have sexual activity during next 72 hrs following the injection.

In accordance to the present invention the injected contraceptive can also be reversed at any desired time for restoration of fertility without any requirement of surgery or flushing of an extra dose of the solvent. Therefore, for reversal of the contraceptive action to restore the fertility, the vas deferens in the scrotal segment is palpated in a special manner to propel the contraceptive preparation into the inguinal segment of the vas deferens. By applying radio frequency diathermy energy from outside the body electrical currents are induced in the electrical conducting particles in the contraceptive causing heating of the contraceptive, softening of the contraceptive and some molecular breakdown. A strong DC electromagnet is rapidly moved over the body surface parallel to the surface marking the spermatic cord, which contains the vas deferens. Alternately, a travelling electromagnetic field is applied and moved over the pelvic region and the procedures are repeated to propel the contraceptive into the ampulla of the vas deferens. Finally with the finger inserted into the rectum via the anus the ampulla of the vas deferens is squeezed to expel the contraceptive into the ejaculatory duct.

In the female the contraceptive is injected into the fallopian tube also known as the oviduct via the ostium. The ostium is the junction of the fallopian tube and the uterus. The site is approached via the vagina and the uterine cervical canal. A hysteroscope is used to visualize the ostium. Since the syringe with the drug is kept outside the body a length of the tubing of about 16 gauge is connected to the syringe. A strong DC permanent magnet or an electromagnet is placed on the abdominal surface such that the field is directed towards the uterus. The field will check the travel of the contraceptive and spillage of the contraceptive into the peritoneal cavity. A hysteroscope is inserted via the vagina and the cervical canal so that the ostium is viewed. The tubing from the syringe is passed via a channel of the hysteroscope so that the tip of the catheter enters the fallopian tube through the ostium. From the syringe the contraceptive is injected into the fallopian tube. Typically 100µl of the contraceptive is injected into one fallopian tube. After injecting into the fallopian tube on one side the hysteroscope is shifted to view the ostium on the other side and advancing the tube the same amount of the contraceptive is injected into the fallopian tube.

In accordance to the present invention the injected contraceptive can also be reversed at any desired time for restoration of fertility without any requirement of surgery or flushing of an extra dose of the solvent. Therefore, for reversal of the contraceptive action to restore the fertility, a radio frequency field is applied to the body to cause induction in the electrical particles and raise the temperature of the particles. A strong DC permanent magnet is moved over the body surface parallel to the fallopian tube so as to propel the contraceptive towards the uterus. Additionally by means of a catheter a suction is applied at the ostium to draw out the contraceptive.

## Claims

1. An injectable reversible contraceptive comprising a contraceptive polymer, a solvent medium, an electrically conducting material and a magnetic material, **characterised in that** said contraceptive polymer is from the hydrogel class of polymers, particularly a mixture of styrene maleic anhydride copolymer and styrene maleic acid copolymer, and said solvent medium is dimethyl sulphoxide solvent, and said electrically conducting material is copper in its pure form essentially consisting of microsize particles and macrosize particles, and said magnetic material is iron in its pure form essentially consisting of microsize particles and macrosize particles.

2. A contraceptive as claimed in claim 1, wherein styrene maleic acid copolymer and styrene maleic anhydride copolymer are taken in the ratio varying between 1.5:8.5 to 3:7, preferably 2:8 with respect to each other.

3. A injectable reversible contraceptive comprising a contraceptive polymer, a solvent medium, an electrically conducting material and a magnetic material, **characterised in that** said contraceptive polymer is from the hydrogel class of polymers, particularly a mixture of styrene maleic anhydride copolymer and styrene maleic acid copolymer, and said solvent medium is dimethyl sulphoxide solvent, and said electrically conducting material is copper in its pure form essentially consisting of microsize particles and macrosize particles, and said magnetic material is iron in the form of oxide or a combination with a biologically accepted material, like sulphur, essentially consisting of microsize particles and macrosize particles.

4. A contraceptive as claimed in claim 1 or 3, wherein said electrically conducting material and said magnetic material each varies between 3 to 20% by weight of said contraceptive polymer.

5. A contraceptive as claimed in claim 4, wherein said electrically conducting material is taken between 3-8%, preferably between 4-6%, more preferably 5% by weight of said contraceptive polymer.

6. A contraceptive as claimed in claim 4, wherein said magnetic material is taken between 6-15%, preferably between 8-12%, more preferably 10% by weight of said contraceptive polymer.

7. A contraceptive as claimed in claim 1 or 3, wherein particle size of said microsize particles of said electrically conducting material is 0.005 to 20µ, preferably 0.5 to 15µ and of said macrosize particles of said electrically conducting material is 150µ to 0.2mm.

8. A contraceptive as claimed in claim 1 or 3, wherein particle size of said microsize particles of magnetic material is 0.005 to 15µ, preferably 0.5 to 15µ and of said macrosize particles of magnetic material is up to 0.5 mm.

9. A contraceptive as claimed in claim 1 or 3, wherein said microsize and macrosize particles of said electrically conducting material are taken in equal amounts by weight.

10. A contraceptive as claimed in claim 1 or 3, wherein said microsize particles of said magnetic material are taken in lower amount as compared to said macrosize particles of said magnetic material.

11. A contraceptive as claimed in claim 1 or 3, wherein for every 100 mg of said contraceptive polymer about 200 µl of said solvent is taken.

12. A contraceptive as claimed in claim 1 or 3, wherein said magnetic material is prevented from aggregation by suitable coating.

13. A contraceptive as claimed in claim 12, wherein said magnetic material is coated with cross-linked styrene maleic anhydride copolymer.

14. A contraceptive as claimed in claim 1 or 3, **characterised in that** the removal of the contraceptive is achieved by external magnetic field, preferably travelling magnetic field or alternately by flushing by another injection of the said solvent.

15. A contraceptive as claimed in claims 1, 3 or 14, **characterised in that** the contraceptive is heated by electromagnetic induction with fields from outside the body, which in-turn causes lowering in viscosity of said contraceptive to effect the reversal thereof.

16. A contraceptive as claimed in claim 1 or 3, **characterised in that** the *in-situ* flow of the contraceptive after injection is controlled by external means, preferably by the application of a drag force or a propelling force by means of an external magnetic field.

17. A contraceptive as claimed in claim 1 or 3, **characterised in that** the presence of the contraceptive is detected and partly quantified by measuring the residual magnetic field strength from outside the body

18. A contraceptive as claimed in claims 1, 3 or 17, **characterised in that** said external means include imaging by ultrasound, X-ray, CAT scan, MRI and scanning electrical impedance plethysmography.

19. A process for preparation of a contraceptive according to claim 1, **characterised by** dissolving the weighed quantities of styrene maleic anhydride copolymer, styrene maleic acid copolymer, said electrically conducting material and said magnetic material in said solvent medium, followed by keeping the complex solution of said copolymers, said electrically conducting material and said magnetic material in an inert environment, preferably in nitrogen atmosphere and shaking for 45-50 hrs by maintaining the temperature at 35°C.

20. A process for preparation of a contraceptive, as claimed in claim 19, wherein said magnetic material is coated magnetic material.

21. A process for preparation of a contraceptive, as claimed in claim 19, wherein said copolymers, and said electrically conducting material and said magnetic material are first mixed and then dissolved in said solvent.

22. A process for preparation of a contraceptive, as claimed in claim 19, wherein said copolymers, and said electrically conducting material and said magnetic material are directly dissolved in said solvent followed by mixing.

23. A process for preparation of a contraceptive, as claimed in claim 19, wherein said copolymers are first mixed and then dissolved in said solvent followed by addition of said electrically conducting material and said magnetic material.

24. A process for preparation of a contraceptive, as claimed in claims 19 and 23, wherein said electrically conducting material and said magnetic material are added either together or one after the other.

## Patentansprüche

1. Ein injizierbares, reversibles Kontrazeptivum, bestehend aus einem kontrazeptiven Polymer, einem Lösungsmedium, einem elektrisch leitenden Stoff und einem magnetischen Stoff, **dadurch gekennzeichnet, dass** das kontrazeptive Polymer ein Polymer aus der Hydrogel-Klasse und genauer gesagt eine Mischung aus Styrol-Maleinsäureanhydrid-Copolymer und Styrol-Maleinsäure-Copolymer ist, dass das Lösungsmedium ein Dimethylsulfoxid-Lösungsmittel ist und dass es sich bei dem elektrisch leitenden Stoff um reines Kupfer handelt, das im Wesentlichen aus Mikropartikeln und Makropartikeln besteht, und dass es sich bei dem magnetischen Stoff um reines Eisen handelt, das im Wesentlichen aus Mikro- und Makropartikeln besteht.

2. Ein Kontrazeptivum nach Anspruch 1, wobei das Verhältnis von Styrol-Maleinsäure-Copolymer zu Styrol-Maleinsäureanhydrid-Copolymer zwischen 1,5:8,5 und 3:7, vorzugsweise jedoch 2:8 beträgt.

3. Ein injizierbares, reversibles Kontrazeptivum, bestehend aus einem kontrazeptiven Polymer, einem Lösungsmedium, einem elektrisch leitenden Stoff und einem magnetischen Stoff, **dadurch gekennzeichnet, dass** das kontrazeptive Polymer ein Polymer aus der Hydrogel-Klasse und genauer gesagt eine Mischung aus Styrol-Maleinsäureanhydrid-Copolymer und Styrol-Maleinsäure-Copolymer ist, dass das Lösungsmedium ein Dimethylsulfoxid-Lösungsmittel ist und dass es sich bei dem elektrisch leitenden Stoffum reines Kupfer handelt, das im Wesentlichen aus Mikropartikeln und Makropartikeln besteht, und dass es sich bei dem magnetischen Stoff um Eisen in Form von Oxid oder einer Kombination mit einem biologisch akzeptablen Stoff wie Schwefel handelt, das im Wesentlichen aus Mikro- und Makropartikeln besteht.

4. Ein Kontrazeptivum nach Anspruch 1 oder 3, wobei die Menge des elektrisch leitenden Stoffs und des magnetischen Stoffs 3 bis 20 Gewichtsprozent des kontrazeptiven Polymers beträgt.

5. Ein Kontrazeptivum nach Anspruch 4, wobei die Menge des elektrisch leitenden Stoffs 3-8 Gewichtsprozent, vorzugsweise 4-6 Gewichtsprozent und noch besser 5 Gewichtsprozent des kontrazeptiven Polymers beträgt.

6. Ein Kontrazeptivum nach Anspruch 4, wobei die Menge des magnetischen Stoffs 6-15 Gewichtsprozent, vorzugsweise 8-12 Gewichtsprozent und noch besser 10 Gewichtsprozent des kontrazeptiven Polymers beträgt.

7. Ein Kontrazeptiwm nach Anspruch 1 oder 3, wobei die Partikelgröße der Mikropartikel des elektrisch leitenden Stoffs 0,005 bis 20 µm und vorzugsweise 0,5 bis 15 µm und die Partikelgröße der Makropartikel des elektrisch leitenden Stoffs 150 µm bis 0,2 mm beträgt.

8. Ein Kontrazeptivum nach Anspruch 1 oder 3, wobei die Partikelgröße der Mikropartikel des magnetischen Stoffs 0,005 bis 15 µm und vorzugsweise 0,5 bis 15 µm und die Partikelgröße der Makropartikel des magnetischen Stoffs bis zu 0,5 mm beträgt.

9. Ein Kontrazeptivum nach Anspruch 1 oder 3, wobei die Mikropartikel und Makropartikel des elektrisch leitenden Stoffs in gleichen Gewichtsmengen vorliegen.

10. Ein Kontrazeptivum nach Anspruch 1 oder 3, wobei die Mikropartikel des magnetischen Stoffs in geringerer Menge als die Makropartikel des magnetischen Stoffs vorliegen.

11. Ein Kontrazeptivum nach Anspruch 1 oder 3, wobei für jeweils 100 mg des kontrazeptiven Polymers etwa 200 µl des Lösungsmittels eingesetzt werden.

12. Ein Kontrazeptivum nach Anspruch 1 oder 3, wobei eine Aggregation des magnetischen Stoffs durch eine geeignete Beschichtung verhindert wird.

13. Ein Kontrazeptivum nach Anspruch 12, wobei der magnetische Stoff mit vernetztem Styrol-Maleinsäureanhydrid-Copolymer beschichtet ist.

14. Ein Kontrazeptivum nach Anspruch 1 oder 3, **dadurch gekennzeichnet, dass** die Entfernung des Kontrazeptivums durch ein externes Magnetfeld erfolgt, vorzugsweise durch ein sich bewegendes Magnetfeld oder durch Ausspülen durch erneute Injektion des Lösungsmittels.

15. Ein Kontrazeptivum nach Anspruch 1, 3 oder 14, **dadurch gekennzeichnet, dass** das Kontrazeptivum durch elektromagnetische Induktion durch Felder außerhalb des Körpers erhitzt wird und dass es durch diese Erhitzung zur Herabsetzung der Viskosität des Kontrazeptivums und damit zur Umkehr seiner Wirkung kommt.

16. Ein Kontrazeptivum nach Anspruch 1 oder 3, **dadurch gekennzeichnet, dass** der In-situ-Fluss des Kontrazeptivums nach der Injektion durch ein externes Mittel gesteuert wird, vorzugsweise durch die Anwendung einer Rücktriebs- oder einer Vortriebskraft mit Hilfe eines externen Magnetfeldes.

17. Ein Kontrazeptivum nach Anspruch 1 oder 3, **dadurch gekennzeichnet, dass** das Vorliegen des Kontrazeptivums durch Messung der verbleibenden magnetischen Feldstärke von außerhalb des Körpers erfasst und teilweise quantifiziert wird.

18. Ein Kontrazeptivum nach Anspruch 1, 3 oder 17, **dadurch gekennzeichnet, dass** dieses externe Mittel eine bildliche Darstellung durch Ultraschall, Röntgen, Computertomographie, Magnetresonanzspektroskopie und Impedanz-Plethysmographie einschließt.

19. Ein Verfahren zur Zubereitung eines Kontrazeptivums nach Anspruch 1, **dadurch gekennzeichnet, dass** die abgewogenen Mengen Styrol-Maleinsäureanhydrid-Copolymer, Styrol-Maleinsäure-Copolymer, elektrisch leitender Stoff und magnetischer Stoff in dem Lösungsmedium gelöst werden und die komplexe Lösung aus diesen Copolymeren, dem elektrisch leitenden Stoff und dem magnetischen Stoff in einer inaktiven Umgebung, vorzugsweise in Stickstoffatmosphäre, aufbewahrt und 45 bis 50 Stunden lang bei einer Temperatur von 35°C geschüttelt werden.

20. Ein Verfahren zur Zubereitung eines Kontrazeptivums nach Anspruch 19, wobei es sich bei dem magnetischen Stoff um einen beschichteten magnetischen Stoff handelt.

21. Ein Verfahren zur Zubereitung eines Kontrazeptivums nach Anspruch 19, wobei die Copolymere, der elektrisch leitende Stoff und der magnetische Stoff zuerst gemischt und dann in dem Lösungsmittel gelöst werden.

22. Ein Verfahren zur Zubereitung eines Kontrazeptivums nach Anspruch 19, wobei die Copolymere, der elektrisch leitende Stoff und der magnetische Stoff direkt in dem Lösungsmittel gelöst und danach gemischt werden.

23. Ein Verfahren zur Zubereitung eines Kontrazeptivums nach Anspruch 19, wobei die Copolymere zuerst gemischt und dann in dem Lösungsmittel gelöst werden und dann der elektrisch leitende Stoff und der magnetische Stoff zugegeben werden.

24. Ein Verfahren zur Zubereitung eines Kontrazeptivums nach Anspruch 19 und 23, wobei der elektrisch leitende Stoff und der magnetische Stoff entweder zusammen oder nacheinander zugegeben werden.

## Revendications

1. Contraceptif réversible injectable comprenant un polymère contraceptif, un milieu solvant, un matériau électroconducteur et un matériau magnétique, **caractérisé en ce que** ledit polymère contraceptif est de la classe des polymères hydrogels, particulièrement un mélange de copolymère styrène-anhydride maléique et de copolymère styrène-acide maléique, et ledit milieu solvant est le solvant diméthylsulfoxyde, et ledit matériau électroconducteur est le cuivre sous sa forme pure consistant essentiellement en microparticules et en macroparticules, et ledit matériau magnétique est le fer dans sa forme pure consistant essentiellement en microparticules et macroparticules.

2. Contraceptif selon la revendication 1, dans lequel le copolymère styrène-acide maléique et le copolymère styrène-anhydride maléique sont pris dans un rapport variant de 1,5:8,5 à 3:7, préférablement de 2:8 l'un par rapport à l'autre.

3. Contraceptif réversible injectable comprenant un polymère contraceptif, un milieu solvant, un matériau électroconducteur et un matériau magnétique, **caractérisé en ce que** ledit polymère contraceptif est de la classe des polymères hydrogels, particulièrement un mélange de copolymère styrène-anhydride maléique et de copolymère styrène-acide maléique, et ledit milieu solvant est le solvant diméthylsulfoxyde, et ledit matériau électroconducteur est le cuivre dans sa forme pure consistant essentiellement en microparticules et en macroparticules, et ledit matériau magnétique est le fer dans sa forme d'oxyde ou dans une combinaison avec un matériau accepté biologiquement, comme le soufre, consistant essentiellement en microparticules et macroparticules.

4. Contraceptif selon la revendication 1 ou 3, dans lequel ledit matériau électroconducteur et ledit matériau magnétique varient chacun entre 3 et 20% en poids dudit polymère contraceptif.

5. Contraceptif selon la revendication 4, dans lequel ledit matériau électroconducteur est pris dans un rapport entre 3-8%, préférablement entre 4-6%, plus préférablement de 5% en poids dudit polymère contraceptif.

6. Contraceptif selon la revendication 4, dans lequel ledit matériau magnétique est pris dans un rapport entre 6-15%, préférablement entre 8-12%, plus préférablement de 10% en poids dudit polymère contraceptif.

7. Contraceptif selon la revendication 1 ou 3, dans lequel la dimension de particules desdites microparticules dudit matériau électroconducteur est 0,005 à 20 µ, préférablement 0,5 à 15 µ et la dimension desdites macroparticules dudit matériau électroconducteur est 150 µ à 0,2 mm.

8. Contraceptif selon la revendication 1 ou 3, dans lequel la dimension de particule desdites microparticules de matériau magnétique est 0,005 à 15 µ, préférablement 0,5 à 15 µ et la dimension desdites macroparticules dudit matériau magnétique peut aller jusqu'à 0,5 mm.

9. Contraceptif selon la revendication 1 ou 3, dans lequel lesdites microparticules et lesdites macroparticules dudit matériau électroconducteur sont prises en quantités égales en poids.

10. Contraceptif selon la revendication 1 ou 3, dans lequel lesdites microparticules dudit matériau magnétique sont prises en quantité plus petite par comparaison auxdites macroparticules dudit matériau magnétique.

11. Contraceptif selon la revendication 1 ou 3, dans lequel pour chaque 100 mg dudit polymère contraceptif il est pris environ 200 µl dudit solvant.

12. Contraceptif selon la revendication 1 ou 3, dans lequel ledit matériau magnétique est empêché de s'agglomérer par un enrobage approprié.

13. Contraceptif selon la revendication 12, dans lequel ledit matériau magnétique est enrobé de copolymère styrène-anhydride maléique réticulé.

14. Contraceptif selon la revendication 1 ou 3, **caractérisé en ce que** l'enlèvement du contraceptif est obtenu par l'emploi d'un champ magnétique extérieur, préférablement un champ magnétique en mouvement ou en variante par le rinçage par une autre injection dudit solvant.

15. Contraceptif selon la revendication 1, 3 ou 14, **caractérisé en ce que** le contraceptif est chauffé par induction électromagnétique avec des champs extérieurs au corps, ce qui à sont tour cause une réduction de la viscosité dudit contraceptif pour obtenir la réaction réversible de celui-ci.

16. Contraceptif selon la revendication 1 ou 3, **caractérisé en ce que** l'écoulement in-situ du contraceptif après l'injection est commandé par un moyen extérieur, préférablement par l'application d'une force de traînée ou d'une force de propulsion au moyen d'un champ magnétique extérieur.

17. Contraceptif selon la revendication 1 ou 3, **caractérisé en ce que** la présence du contraceptif est détectée et quantifiée partiellement en mesurant la force du champ magnétique de l'extérieur du corps.

18. Contraceptif selon la revendication 1, 3 ou 17, **caractérisé en ce que** ledit moyen extérieur comprend la formation d'images par ultrasons, rayons X, IRM et pléthysmographie à balayage à impédance électrique.

19. Procédé de préparation d'un contraceptif selon la revendication 1, **caractérisé par** la dissolution de quantités pesées de copolymère styrène-anhydride maléique, de copolymère styrène-acide maléique, dudit matériau électroconducteur et dudit matériau magnétique dans ledit milieu solvant, suivi par la conservation de la solution complexe desdits copolymères, dudit matériau électroconducteur et dudit matériau magnétique dans un environnement inerte, préférablement dans une atmosphère d'azote avec agitation pendant 45-50 h en maintenant la température à 35°C.

20. Procédé de préparation d'un contraceptif selon la revendication 19, dans lequel ledit matériau magnétique est un matériau magnétique enrobé.

21. Procédé de préparation d'un contraceptif selon la revendication 19, dans lequel lesdits copolymères et ledit matériau électroconducteur et ledit matériau magnétique sont premièrement mélangés et ensuite dissous dans ledit solvant.

22. Procédé de préparation d'un contraceptif selon la revendication 19, dans lequel lesdits copolymères et ledit matériau électroconducteur et ledit matériau magnétique sont dissous directement dans ledit solvant et ensuite mélangés.

23. Procédé de préparation d'un contraceptif selon la revendication 19, dans lequel lesdits copolymères sont premièrement mélangés et ensuite dissous dans ledit solvant, suivi par l'addition dudit matériau électroconducteur et dudit matériau magnétique.

24. Procédé de préparation d'un contraceptif selon les revendications 19 et 23, dans lequel ledit matériau électroconducteur et ledit matériau magnétique sont ajoutés soit ensemble, soit l'un après l'autre.
